## Europäisches Patentamt

## European Patent Office

## Office européen des brevets

(19)

(11) Veröffentlichungsnummer: **0 212 308**
**B1**

(12) **EUROPÄISCHE PATENTSCHRIFT**

(45) Veröffentlichungstag der Patentschrift: **04.07.90**

(21) Anmeldenummer: **86110132.7**

(22) Anmeldetag: **23.07.86**

(51) Int. Cl.⁵: **A 61 B 17/32,** A 61 M 1/00

(54) Arthroskopie-Instrument mit Absaugrohr.

(30) Priorität: **26.07.85 DE 3526822**

(43) Veröffentlichungstag der Anmeldung:
**04.03.87 Patentblatt 87/10**

(45) Bekanntmachung des Hinweises auf die
Patenterteilung:
**04.07.90 Patentblatt 90/27**

(84) Benannte Vertragsstaaten:
**AT BE CH DE FR GB IT LI LU NL SE**

(56) Entgegenhaltungen:
**EP-A-0 119 405**
**DE-B-1 091 700**
**DE-C- 932 741**
**GB-A-1 215 439**
**US-A-2 751 908**

(73) Patentinhaber: **Hensler, Ewald**
**Hegaustrasse 3**
**D-7717 Hattingen (DE)**

(72) Erfinder: **Hensler, Ewald**
**Hegaustrasse 3**
**D-7717 Hattingen (DE)**

(74) Vertreter: **Heusler, Wolfgang, Dipl.-Ing. et al**
**Dr. Dieter von Bezold Dipl.-Ing. Peter Schütz**
**Dipl.-Ing. Wolfgang Heusler Brienner Strasse 52**
**D-8000 München 2 (DE)**

Courier Press, Leamington Spa, England.

## Beschreibung

Die vorliegende Erfindung betrifft ein Arthroskopie-Instrument gemäß dem Oberbegriff des Anspruchs 1.

Ein Arthroskopie-Instrument dieser Art ist aus der EP-A-0119405 bekannt.

Bei dem bekannten Instrument soll das Betätigungsglied für das bewegliche Stanzenteil aus einem verschiebbar in einem Außenrohr gelagerten, selbst als Saugrohr dienenden Innenrohr bestehen. In der genannten Druckschrift wird erwähnt, daß das Betätigungsglied stattdessen auch aus einer massiven Stange bestehen kann, die in dem als Saugrohr dienenden Außenrohr nahe an dessen Innenwand angeordnet ist, doch fehlen weitere Hinweise für eine konstruktive Realisierung dieser Alternativmöglichkeit.

Es ist ferner aus US-A-2751908 ein Arthroskopie-Instrument bekannt, das sich von dem oben beschriebenen Instrument hauptsächlich dadurch unterscheidet, daß eine massive Schubstange in einer sie eng umgebenden Führungsrohr gelagert ist, an dessen vorderem Ende eine hufeisenartige, seitlich vorstehende Schneide angebracht ist, welche mit einem durch die Schubstange betätigten um die Achse der Schubstange schwenkbaren Stanzenteil zusammenwirkt. Der Absaugkanal wird durch ein eigenes Rohr gebildet, welches neben dem Führungsrohr verläuft und mit diesem verbunden ist. Dieses Instrument hat den Nachteil, daß sein Arbeitsende keine glatte Außenfläche hat und ein sicheres Absaugen von abgetrennten Knorpelstücken nicht gewährleistet ist.

Der Erfindung liegt die Aufgabe zugrunde, ein Arthroskopielnstrument der ersterwähnten Art zu schaffen, dessen Betätigungsstange sicher ohne Gefahr einer Verstopfung des Absaugkanals durch abgeschnittene Knorpelstücke gelagert werden kann, und das gut zu reinigen ist.

Diese Aufgabe wird durch das im Patentanspruch 1 gekennzeichnete Arthroskopie-Instrument gelöst. Weiterbildungen und vorteilhafte Ausgestaltungen des erfindungsgemäßen Arthroskopie-Instruments sind Gegenstand der Unteransprüche.

Bei dem vorliegenden Arthroskopie-Instrument nehmen die Betätigungsstange und ihre Lagerung nur einen relativ kleinen Teil des Innenquerschnitts des Saugrohres ein. Es steht also ein großer Querschnitt für das Absaugen von abgeschnittenen Knorpelteilen zur Verfügung. Wird das Maulteil gemäß einer vorteilhaften Ausgestaltung des vorliegenden Arthroskopie-Instruments so gestaltet, daß der abgeschnittene Knorpelteil erheblich kleiner ist als der freie Raumquerschnitt des Absaugrohres, so ist eine schnelle und sichere Absaugung der abgetrennten Knorpelteile gewährleistet. Das Maulteil wird ferner vorzugsweise so gestaltet, daß abgeschnittene Knorpelteile in den sich unterhalb des Stanzenteiles befindenden Raum abwandern und sich dort relativ frei entspannen können, so daß sie dann ungehindert abgesaugt werden können. Abgeschnittene Knorpelteile können sich an den Lagerstellen nicht festsetzen.

Die Lagerstellen können zweckmäßigerweise so ausgestaltet sein, daß die durch sie bewirkte Querschnittsverkleidung kleiner ist als die durch die Betätigungsstangte. Sie haben vorzugsweise eine radiale Abmessung, die kleiner ist als der Durchmesser der Betätigungsstange. Die Lagerstellen sind gut zu reinigen, z.B. mit einer Bürste, und da sie sehr reibungsarm sind, bleibt das Instrument stets leichtgängig.

Im folgenden wird eine bevorzugte Ausführungsform der Erfindung unter Bezugnahme auf die Zeichnungen näher erläutert.

Es zeigen:

Fig. 1 eine Seitenansicht einer bevorzugten Ausführungsform des Arthroskopie-Instruments gemäß der Erfindung;

Fig. 2 eine vergrößerte Schnittansicht des Maulteils;

Fig. 3 einen Querschnitt in einer Ebene A-B der Fig. 1; und

Fig. 4 eine Querschnittsansicht eines Teiles der Betätigungsvorrichtung.

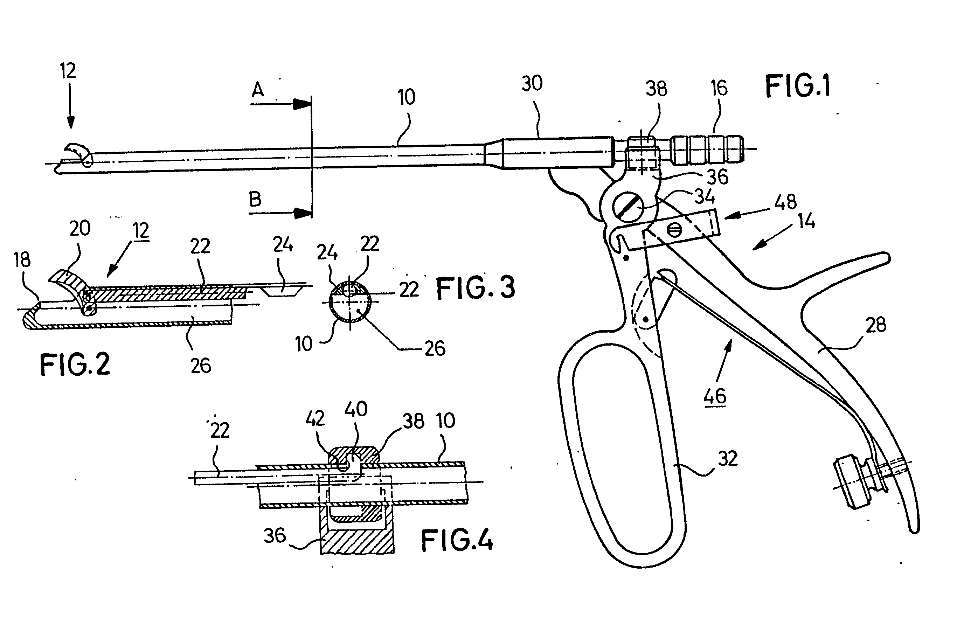

Das in der Zeichnung dargestellte Arthroskopie-Instrument enthält ein Saugrohr 10, das am vorderen Ende ein nahtlos angeschweißtes Maulteil 12 (Fig. 2) aufweist. Am hinteren Ende befindet sich eine Betätigungsvorrichtung 14 sowie ein Anschlußstück 16 für eine Absaugleitung.

Das Maulteil hat ein rundes geschlossenes Vorderende mit einer seitlichen Öffnung, deren Rand eine Schneide 18 bildet, welche mit einem im Saugrohr schwenkbar gelagerten Stanzenteil 20 zusammenwirkt. Das Stanzenteil 20 ist an einer massiven Betätigungsstange 22 angelenkt, deren Durchmesser wesentlich kleiner als der Innendurchmesser des Saugrohres 10 ist, z.B. kleiner als ein Drittel des Saugrohr-Innendurchmessers. Die Betätigungsstange 22 ist exzentrisch an der Innenwand des Saugrohres angeordnet und durch z. B. drei Lagerstellen 24 längsverschieblich gelagert. Die Lagerstellen 24 haben die Form von Kreissegmenten, die in das Saugrohr passen und bei der dargestellten bevorzugten Ausführungsform radial nicht so weit in das Innere des Saugrohres reichen wie die Betätigungsstange 22, so daß ein Saugkanal 26 großen Querschnitts frei bleibt. Die axialen Enden der Lagerstücke 24 können abgeschrägt sein, wie es in Fig. 2 dargestellt ist, so daß die Lagerstücke einen etwa trapezförmigen Querschnitt haben, durch den ein Festsetzen von abgeschnittenen Knorpelteilen sicher verhindert wird.

Das Maulteil 12 ist so gestaltet, daß abgeschnittene Knorpelteile in den unteren Teil abwandern, sich dort entspannen (ausdehnen) und dann ungehindert abgesaugt werden können. Das Maulteil ist ferner vorteilhafterweise so bemessen, daß die jeweils abgeschnittenen Knorpelteile erheblich kleiner sind als der freie Raumquerschnitt des Absaugkanals 26.

Die Betätigungsvorrichtung 14 ist nach Art eines Scherengriffes ausgebildet und enthält ein erstes Griffstück 28, das über eine Halterungsmuffe 30 fest mit dem Saugrohr 10 verbunden ist. Ferner enthält die Betätigungsvorrichtung ein zweites Griffstück 32, welches durch ein Drehlager 34 mit dem ersten Griffstück 28 schwenkbar verbunden ist und an dem über das Drehlager 34 hinausreichenden Teil ein gabelförmiges Ende 36 aufweist, das mit einer Buchse 38 in Eingriff steht, die mit engem Gleitsitz auf dem Saugrohr 10 sitzt. Das dem Maulteil abgewandte Ende der Betätigungsstange 22 hat, wie Fig. 4 zeigt, ein rechtwinklig umgebogenes Ende 40, welches durch ein im Saugrohr 10 vorgesehenes, in dessen Längsrichtung verlaufendes Langloch 42 nach außen reicht und in eine innere Ausnehmung der Muffe 38 eingreift. Durch Schwenken des Griffstückes 32 wird die Muffe 38 und damit die Betätigungsstange 22 in Längsrichtungs des Saugrohres verschoben und letztlich das Stanzteil 20 betätigt. Die Muffe 38 liegt dicht an der Außenseite des Saugrohrs 10 an und dichtet das Langloch 42 ausreichend ab. Ihre axiale Abmessung ist so groß, daß das Langloch 42 in allen Stellungen, denen die Muffe 38 fähig ist, abgedichtet bleibt.

Die Betätigungsvorrichtung 14 ist mit einer üblichen Federanordnung 46 versehen, die die Griffstücke 28, 32 auseinanderzudrücken und dadurch das Maulteil zu öffnen strebt. Ferner ist eine Arretiervorrichtung 48 vorgesehen, mit der die Griffstücke im zusammengedrückten Zustand, in dem das Mauteil geschlossen ist, arretiert werden kann.

Das Saugrohr 10 kann einen üblichen Außendurchmesser von 5,5 mm oder weniger aufweisen. Das Maulteil kann den Erfordernissen entsprechend im Winkel am Saugrohr ansetzen. Alle Teile des Instruments bestehen aus nichtrostendem Edelstahl, so daß jede Art der Reinigung oder Sterilisierung durchgeführt werden kann.

Durch die Erfindung wird also ein funktionssicheres, stabiles Instrument geschaffen, dessen Saugrohr nahtlos und ohne Querschnittsverengung in das Maulteil übergeht.

**Patentansprüche**

1. Arthroskopie-Instrument mit einem Saugrohr (10), welches am vorderen Ende ein Maulteil (12) hat, das eine Schneide (18) und ein mit dieser zusammenwirkendes, am Maulteil (12) angelenktes Stanzenteil (20) enthält, und welches am hinteren Ende mit einer Betätigungsvorrichtung (14) verbunden ist, die ein erstes, am Saugrohr (10) fest angebrachtes Griffstück (28) und ein zweites, bezüglich des Saugrohres bewegliches Griffstück (32) enthält, und mit einer exzentrisch im Saugrohr (10) bei dessen Innenwand längsverschieblich gelagerten Betätigungsstange (22), welche das bewegliche Stanzenteil (20) mit dem beweglichen Griffstück (32) koppelt und einen Außendurchmesser hat, der wesentlich kleiner ist als der Innendurchmesser des Saugrohres, so daß im Saugrohr ein Absaugkanal (26) frei bleibt, der bei der Betätigungsvorrichtung (14) mit einem Anschlußstück (16) versehen ist, dadurch gekennzeichnet, daß die Betätigungsstange (22) an der Innenwand des Saugrohrs (10) durch eine Anzahl im Abstand voneinander angeordneter Halterungsklauen (24) gehaltert ist, welche die Form von in das Saugrohr (10) passenden Kreissegmenten mit einem Loch für die Betätigungsstange (22) haben, radial höchstens so weit in das Innere des Saugrohres (10) hineinreichen wie die Betätigungsstange (22) und abgeschrägte axiale Enden haben (24, Fig. 2).

2. Arthroskopie-Instrument nach Anspruch 1, dadurch gekennzeichnet, daß die Halterungsklauen (24) radial weniger weit in das Innere des Saugrohres (10) hineinreichen als die Betätigungsstange (22).

3. Arthroskopie-Instrument nach einem der vorhergehenden Ansprüche, dadurch gekennzeichnet, daß der Durchmesser der Betätigungsstange (22) kleiner als ein Drittel des Innendurchmessers des Saugrohres (10) ist.

4. Arthroskopie-Instrument nach einem der vorhergehenden Ansprüche, dadurch gekennzeichnet, daß das dem Maulteil (12) abgewandte Ende (40) der Betätigungsstange (22) umgebogen ist, durch einen longitudinalen Schlitz (42) im Saugrohr (10) nach außen reicht und in eine den Schlitz abdichtende, auf der Außenseite des Saugrohres bei der Betätigungsvorrichtung (14) verschiebbar gelagerte Muffe (38) eingreift, welche mit dem beweglichen Griffstück (32) gekoppelt und durch dieses verschiebbar ist.

5. Arthroskopie-Instrument nach Anspruch 4, dadurch gekennzeichnet, daß die Griffstücke (28, 32) nach Art von Scherengriffen ausgebildet und durch ein Drehlager (34) miteinander verbunden sind und daß das bewegliche Griffstück (32) ein über das Drehlager hinausreichendes gabelartiges Ende (36) aufweist, welches mit der Muffe (38) in Eingriff steht.

6. Arthroskopie-Instrument nach einem der vorhergehenden Ansprüche, dadurch gekennzeichnet, daß das Maulteil (12) so gestaltet ist, daß jeder abgeschnittene Knorpelteil erheblich kleiner ist als der freie Raumquerschnitt des Saugkanals (26).

**Revendications**

1. Instrument d'arthroscopie, comportant un tube d'aspiration (10), qui présente à l'extrémité avant une partie d'embouchure (12), contenant une lame (18) et une partie de découpage (20) coopérant avec celle-ci et articulée sur la partie d'embouchure (12), reliée à l'extrémité arrière à un dispositif d'actionnement (14), qui contient une première pièce de prise (28) montée rigidement sur le tube d'aspiration (10), et une deuxième pièce de prise (32), mobile par rapport au tube d'aspiration, et avec une tige d'actionnement (22), montée excentrique sur le tube d'aspiration (10) et déplaçable longitudinalement sur sa paroi intérieure, qui accouple la partie de découpage (20) mobile à la pièce de prise (32) mobile et présente un diamètre extérieur qui est sensiblement inférieur du tube d'aspiration, de façon qu'un canal d'aspiration (26), qui est pourvu d'une pièce de raccordement (16) sur le dispositif d'actionnement (14), reste libre dans le tube d'aspiration, caractérisé en ce que la tige d'actionnement (22) est maintenue sur la paroi intérieure du tube d'aspiration (10) par un certain nombre de griffes de fixation (24) disposées à distance les unes des autres, qui ont la forme de segments de cercle s'adaptant dans le tube d'aspiration (10), avec un trou pour la tige d'actionnement (22), qui arrivent radialement au plus aussi loin que la tige d'actionnement (22) à l'intérieur du tube d'aspiration (10) et qui présentent des extrémités chanfreinées axialement (24, figure 2).

2. Instrument d'arthroscopie selon la revendication 1, caractérisé en ce que les griffes de fixation (24) arrivent radialement moins loin que la tige d'actionnement (22) à l'intérieur du tube d'aspiration (10).

3. Instrument d'arthroscopie selon l'une des revendications précédentes, caractérisé en ce que le diamètre de la tige d'actionnement (22) est inférieur au tiers du diamètre intérieur du tube d'aspiration (10).

4. Instrument d'arthroscopie selon l'une des revendications précédentes, caractérisé en ce que l'extrémité (42) de la tige d'actionnement (22) qui est opposée à la partie d'embouchure (12) est recourbée, qu'elle passe à l'extérieur à travers une fente (42) longitudinale ménagée dans le tube d'aspiration (10) et s'engage dans un manchon (38), assurant l'étanchéité de la fente et monté déplaçable sur le dispositif d'actionnement (14) sur la face extérieure du tube d'aspiration, qui est accouplé à la pièce de prise (32) mobile est susceptible d'être déplacé au moyen de celle-ci.

5. Instrument d'arthroscopie selon la revendication 4, caractérisé en ce que les pièces de prise (28, 32) sont réalisés à la manière de poignées de ciseaux et reliées entre elles par un palier tournant (34), et en ce que la pièce de prise mobile (32) présente une extrémité (36) en fourchette, passant au-dessus du pallier tournant, qui vient au contact du manchon (38).

6. Instrument d'arthroscopie selon l'une des revendications précédentes, caractérisé en ce que la partie d'embouchure (12) est configurée de façon que chaque partie de cartilage découpée soit sensiblement plus petite que la section transversale d'espace libre du canal d'aspiration.

## Claims

1. Arthroscopic instrument including a suction tube (10) which at its front end has a jaw part (12) which includes a cutting blade (18) and a die part (20) cooperating with it and pivotally connected to the jaw part (12) and which at its rear end is connected to an actuating device (14) which includes a first handle member (28) rigidly mounted on the suction tube (10) and a second handle member (32) which is movable with respect to the suction tube, and including an actuating rod (22) which is longitudinally movably and eccentrically mounted in the suction tube (10) adjacent its inner wall and which couples the movable die part (20) to the movable handle member (32) and has an external diameter which is substantially smaller than the internal diameter of the suction tube so that a suction passage (26), which is provided with a connecting member (16) adjacent the actuating device (14), remains clear within the suction tube, characterised in that the actuating rod (22) is retained at the inner wall of the suction tube (10) by a number of spaced retaining claws (24) which have the shape of circular segments, fitting into the suction tube (10) with a hole for the actuating rod (22), extend radially into the interior of the suction tube (10) at most as far as the actuating rod (22) and have bevelled axial ends (24, Fig. 2).

2. Arthroscopic instrument as claimed in claim 1, characterised in that the retaining claws (24) extend less far radially into the interior of the suction tube (10) than the actuating rod (22).

3. Arthroscopic instrument as claimed in one of the preceding claims, characterised in that the diameter of the actuating rod (22) is less than one third of the internal diameter of the suction tube (10).

4. Arthroscopic instrument as claimed in one of the preceding claims, characterised in that the end (40) of the actuating rod (22) remote from the jaw part (12) is bent back on itself, extends outwardly through a longitudinal slot (42) in the suction tube (10) and engages in a sleeve (38) which seals the slot and is movably mounted on the exterior of the suction tube adjacent the actuating device (14) and which is coupled to the movable handle member (32) and may be moved by it.

5. Arthroscopic instrument as claimed in claim 4, characterised in that the handle members (28,32) are constructed in the manner of scissor handles and are connected together by a pivotal bearing (34) and that the movable handle member (32) has a bifurcated end (36) which extends beyond the pivotal bearing and which is in engagement with the sleeve (38).

6. Arthroscopic instrument as claimed in one of the preceding claims, characterised in that the jaw part (12) is so constructed that each portion of cartilage which is cut off is considerably smaller than the free cross-section of the suction passage (26).

FIG.1

FIG.2

FIG.3

FIG.4

EP 0 212 308 B1